(19) Europäisches Patentamt
European Patent Office
Office européen des brevets

(11) Veröffentlichungsnummer: **0 608 709 A1**

# (12) EUROPÄISCHE PATENTANMELDUNG

(21) Anmeldenummer: **94100366.7**

(22) Anmeldetag: **12.01.94**

(51) Int. Cl.[5]: **C07D 215/22**, A61K 31/47, C07D 401/10

(30) Priorität: **25.01.93 DE 4301900**

(43) Veröffentlichungstag der Anmeldung: **03.08.94 Patentblatt 94/31**

(84) Benannte Vertragsstaaten: **AT BE CH DE DK ES FR GB GR IE IT LI LU MC NL PT SE**

(71) Anmelder: **BAYER AG**

**D-51368 Leverkusen(DE)**

(72) Erfinder: **Müller-Gliemann, Matthias, Dr.**
**Laibacher Strasse 10**
**D-42697 Solingen(DE)**
Erfinder: **Dressel, Jürgen, Dr.**
**Tuchstrasse 48**
**D-42477 Radevormwald(DE)**
Erfinder: **Fey, Peter, Dr.**
**Am Eickhof 23**
**D-42111 Wuppertal(DE)**
Erfinder: **Hanko, Rudolf, Dr.**
**Schillerstrasse 23**
**D-40237 Düsseldorf(DE)**
Erfinder: **Hübsch, Walter, Dr.**
**Wildsteig 22**
**D-42113 Wuppertal(DE)**
Erfinder: **Krämer, Thomas, Dr.**
**Schneewittchenweg 37**
**D-42111 Wuppertal(DE)**
Erfinder: **Müller, Ulrich E., Dr.**
**Neuer Triebel 91**
**D-42111 Wupppertal(DE)**
Erfinder: **Raddatz, Siegfried, Dr.**
**Jakob-Böhme-Strasse 21**
**D-51065 Köln(DE)**
Erfinder: **Beuck, Martin, Dr.**
**Trills 7**
**D-40699 Erkrath(DE)**
Erfinder: **Kazda, Stanislav, Prof. Dr.**
**Gellertweg 18**
**D-42115 Wuppertal(DE)**
Erfinder: **Wohlfeil, Stefan, Dr.**
**Tucherweg 25**
**D-40724 Hilden(DE)**
Erfinder: **Knorr, Andreas, Dr.**
**Trillser Graben 10**
**D-40699 Erkrath(DE)**
Erfinder: **Stasch, Johannes-Peter, Dr.**
**Alfred-Nobel-Strasse 109**
**D-42651 Solingen(DE)**
Erfinder: **Zaiss, Siegfried, Dr.**
**Farnweg 3**
**D-42113 Wuppertal(DE)**

## (54) 2-Oxochinolin-l-yl-methyl-phenylessigsäurederivate als Angiotensin II Antagonisten.

(57) 2-Oxochinolin-1-yl-methylphenylessigsäurederivate wurden hergestellt durch Umsetzung von 2-Hydroxychinolinen mit entsprechend substituierten Benzylbromiden. Die Verbindungen eignen sich als Wirkstoffe in Arzneimitteln zur Behandlung von arterieller Hypertonie und Atherosklerose.

EP 0 608 709 A1

Die Erfindung betrifft 2-Oxochinolin-1-yl-methylphenylessigsäurederivate, Verfahren zu ihrer Herstellung sowie ihre Verwendung in Arzneimitteln, insbesondere als blutdrucksenkende und antiatherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Die vorliegende Erfindung betrifft 2-Oxochinolin-1-yl-methylphenylessigsäurederivate der allgemeinen Formel (I)

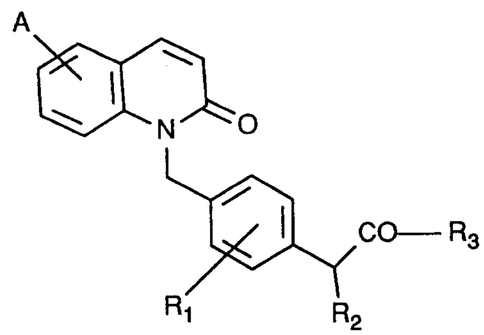

(I)

in welcher

A und $R^1$ gleich oder verschieden sind und für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano, Carboxy, Cycloalkyl mit 3 bis 8 Kohlenstoffatomen oder Phenyl stehen,

$R^2$ für Wasserstoff, Cycloalkyl mit 3 bis 8 Kohlenstoffatomen oder für geradkettiges oder verzweigtes Alkyl mit bis zu 8 Kohlenstoffatomen steht, das gegebenenfalls durch Cycloalkyl mit 3 bis 8 Kohlenstoffatomen substituiert ist,

$R^3$ für Hydroxy oder geradkettiges oder verzweigtes Alkoxy mit bis zu 8 Kohlenstoffatomen steht, oder für eine Gruppe der Formel $-NR^4R^5$ ,

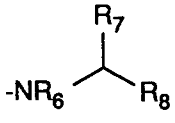

oder

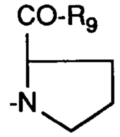

steht,

worin

$R^4$, $R^5$ und $R^6$ gleich oder verschieden sind und Wasserstoff, geradkettiges oder verzweigtes Alkyl mit bis zu 6 Kohlenstoffatomen oder Phenyl bedeuten,

oder

$R^4$ die oben angegebene Bedeutung hat

und

$R^5$ eine Gruppe der Formel $-SO_2-R^{10}$ bedeutet,

worin

$R^{10}$ geradkettiges oder verzweigtes Alkyl mit bis zu 6 Kohlenstoffatomen bedeutet, das gegebenenfalls durch Phenyl substituiert ist, oder Trifluormethyl bedeutet oder

Phenyl bedeutet, das gegebenenfalls durch geradkettiges oder verzweigtes Alkyl mit bis zu 4 Kohlenstoffatomen substituiert ist,

$R^7$ Phenyl bedeutet, das gegebenenfalls bis zu 2-fach gleich oder verschieden durch Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano, Carboxy, Cycloalkyl mit 3 bis 8 Kohlenstoffatomen oder Phenyl substituiert ist,

$R^8$ Carboxy oder geradkettiges oder verzweigtes Alkoxycarbonyl mit bis zu 6 Kohlenstoffatomen bedeutet, oder

eine Gruppe der Formel $-CH_2-OR^{11}$, $-CO-NR^{12}R^{13}$ oder $-CH_2NR^{12}R^{13}$ bedeutet,

worin

$R^{11}$ Wasserstoff, Phenyl oder geradkettiges oder verzweigtes Alkyl mit bis zu 6 Kohlenstoffatomen bedeutet

und

$R^{12}$ und $R^{13}$ die oben angegebene Bedeutung von $R^4$ und $R^5$ haben und mit diesen gleich oder verschieden sind,

$R^9$ Hydroxy, Benzyloxy oder geradkettiges oder verzweigtes Alkoxy mit bis zu 8 Kohlenstoffatomen bedeutet, oder

eine Gruppe der Formel

$-NR^{14}R^{15}$ oder

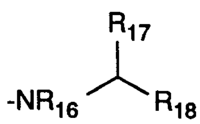

bedeutet,

worin

$R^{14}$ und $R^{15}$ die oben angegebene Bedeutung von $R^4$ und $R^5$ haben und mit dieser gleich oder verschieden sind,

$R^{16}$ die oben angegebene Bedeutung von $R^6$ hat und mit dieser gleich oder verschieden ist,

$R^{17}$ und $R^{18}$ jeweils die oben angegebene Bedeutung von $R^7$ bzw. $R^8$ haben und mit diesen gleich oder verschieden sind,

gegebenenfalls in einer isomeren Form und deren Salze.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der 2-Oxochinolin-1-y-methylphenylessigsäurederivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren, Diastereomeren als auch deren jeweiligen Mischungen. Die Racemformen lassen sich ebenso wie die Diastereomeren in bekannter Weise in die

stereoisomer einheitlichen Bestandteile trennen.

Bevorzugt sind Verbindungen der allgemeinen Formel (I), in welcher

| | |
|---|---|
| A und $R^1$ | gleich oder verschieden sind und für Wasserstoff, Fluor, Chlor, Brom, Trifluormethyl, Cyano oder geradkettiges oder verzweigtes Alkyl, Alkoxy oder Alkoxycarbonyl mit jeweils bis zu 4 Kohlenstoffatomen stehen, |
| $R^2$ | für Wasserstoff, Cyclopropyl, Cyclopentyl, Cyclohexyl oder Cycloheptyl steht, oder für geradkettiges oder verzweigtes Alkyl mit bis zu 6 Kohlenstoffatomen steht, das gegebenenfalls durch Cyclopentyl, Cyclohexyl oder Cycloheptyl substituiert ist, |
| $R^3$ | für Hydroxy oder geradkettiges oder verzweigtes Alkoxy mit bis zu 6 Kohlenstoffatomen steht, oder für eine Gruppe der Formel $-NR^4R^5$ , 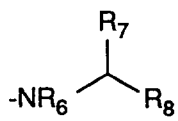 oder 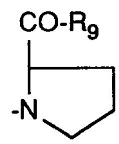 steht, worin |
| $R^4$, $R^5$ und $R^6$ | gleich oder verschieden sind und Wasserstoff oder geradkettiges oder verzweigtes Alkyl mit bis zu 4 Kohlenstoffatomen bedeuten, oder |
| $R^4$ | die oben angegebene Bedeutung hat und |
| $R^5$ | eine Gruppe der Formel $-SO_2-R^{10}$ bedeutet, worin |
| $R^{10}$ | geradkettiges oder verzweigtes Alkyl mit bis zu 4 Kohlenstoffatomen bedeutet, das gegebenenfalls durch Phenyl substituiert ist, oder Trifluormethyl bedeutet, oder Phenyl bedeutet, das gegebenenfalls durch geradkettiges oder verzweigtes Alkyl mit bis zu 3 Kohlenstoffatomen substituiert ist, |
| $R^7$ | Phenyl bedeutet, das gegebenenfalls bis zu 2-fach gleich oder verschieden durch Fluor, Chlor, Brom, Trifluormethyl, Carboxy, geradkettiges oder verzweigtes Alkyl, Alkoxy oder Alkoxycarbonyl mit jeweils bis zu 4 Kohlenstoffatomen, Cyclopentyl, Cyclohexyl oder Phenyl substituiert ist, |
| $R^5$ | Carboxy oder geradkettiges oder verzweigtes Alkoxycarbonyl mit bis zu 4 Kohlenstoffatomen bedeutet, oder eine Gruppe der Formel $-CH_2-OR^{11}$, $-CO-NR^{12}R^{13}$ oder $-CH_2NR^{12}R^{13}$ bedeutet, worin |
| $R^{11}$ | Wasserstoff oder geradkettiges oder verzweigtes Alkyl mit bis zu 4 Kohlenstoffatomen bedeutet und |
| $R^{12}$ und $R^{13}$ | die oben angegebene Bedeutung von $R^4$ und $R^5$ haben und mit dieser gleich oder verschieden sind, |
| $R^9$ | Hydroxy, Benzyloxy oder geradkettiges oder verzweigtes Alkoxy mit bis zu 6 Kohlenstoffatomen bedeutet, oder eine Gruppe der Formel $-NR^{14}R^{15}$ oder 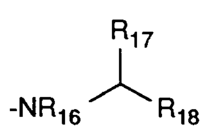 |

bedeutet,
worin

$R^{14}$ und $R^{15}$ die oben angegebene Bedeutung von $R^4$ und $R^5$ haben und mit dieser gleich oder verschieden sind,

$R^{16}$ die oben angegebene Bedeutung von $R^6$ hat und mit dieser gleich oder verschieden ist,

$R^{17}$ und $R^{18}$ jeweils die oben angegebene Bedeutung von $R^7$ bzw. $R^8$ haben und mit diesen gleich oder verschieden sind,

gegebenenfalls in einer isomeren Form und deren Salze.

Besonders bevorzugt sind Verbindungen der allgemeinen Formel (I),
in welcher

A und $R^1$ gleich oder verschieden sind und
für Wasserstoff, Fluor, Chlor, Brom, Trifluormethyl, Trifluormethoxy, Cyano oder Methyl stehen,

$R^2$ für Wasserstoff, Cyclopropyl, Cyclopentyl, Cyclohexyl, Cycloheptyl oder für geradkettiges oder verzweigtes Alkyl steht, das gegebenenfalls durch Cyclopentyl oder Cyclohexyl substituiert ist,

$R^3$ für Hydroxy oder geradkettiges oder verzweigtes Alkoxy mit bis zu 4 Kohlenstoffatomen steht, oder
für eine Gruppe der Formel
$-NR^4R^5$ ,

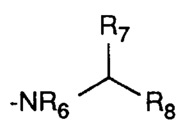

oder

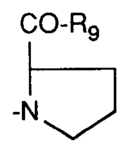

steht,
worin

$R^4$, $R^5$ und $R^6$ gleich oder verschieden sind und Wasserstoff oder Methyl bedeuten
oder

$R^4$ die oben angegebene Bedeutung hat
und

$R^5$ eine Gruppe der Formel $-SO_2-R^{10}$ bedeutet,
worin

$R^{10}$ Methyl, Benzyl oder p-Tolyl bedeutet,

$R^7$ Phenyl bedeutet, das gegebenenfalls durch Fluor, Chlor, Brom, Trifluormethyl, Trifluormethoxy oder Methyl substituiert ist,

$R^8$ Carboxy oder geradkettiges oder verzweigtes Alkoxycarbonyl mit bis zu 3 Kohlenstoffatomen bedeutet, oder
eine Gruppe der Formel $-CH_2O-R^{11}$, $-CO-NR^{12}R^{13}$ oder $-CH_2NR^{12}R^{13}$ bedeutet,
worin

$R^{11}$ Wasserstoff oder geradkettiges oder verzweigtes Alkyl mit bis zu 3 Kohlenstoffatomen bedeutet
und

$R^{12}$ und $R^{13}$ die oben angegebene Bedeutung von $R^4$ und $R^5$ haben und mit dieser gleich oder verschieden sind,

$R^9$ Hydroxy, Benzyloxy oder geradkettiges oder verzweigtes Alkoxy mit bis zu 4 Kohlenstoffatomen bedeutet, oder
eine Gruppe der Formel
$-NR^{14}R^{15}$ oder

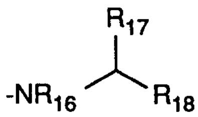

bedeutet,
worin

$R^{14}$ und $R^{15}$ die oben angegebene Bedeutung von $R^4$ und $R^5$ haben und mit dieser gleich oder verschieden sind,

$R^{16}$ die oben angegebene Bedeutung von $R^6$ hat und mit dieser gleich oder verschieden ist,

$R^{17}$ und $R^{18}$ jeweils die oben angegebene Bedeutung von $R^7$ bzw. $R^8$ haben und mit diesen gleich oder verschieden sind,

gegebenenfalls in einer isomeren Form und deren Salze.

Ganz besonders bevorzugt sind Verbindungen der allgemeinen Formel (I), in welcher

A und $R^1$ für Wasserstoff stehen,

$R^2$ für Cyclopentyl oder Cycloheptyl steht

und

$R^3$ für Hydroxy, geradkettiges oder verzweigtes Alkoxy mit bis zu 4 Kohlenstoffatomen oder
für eine Gruppe der Formel

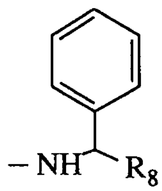

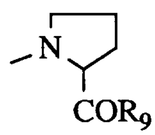

steht,
wobei

$R^8$ die $-CONH_2$-Gruppe bedeutet,

$R^9$ Hydroxy, geradkettiges oder verzweigtes Alkoxy mit bis zu 4 Kohlenstoffatomen bedeutet,
oder eine Gruppe der Formel
$-NR^{14}R^{15}$ oder

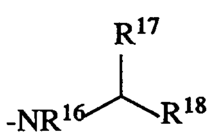

bedeutet,
worin

$R^{14}$, $R^{15}$, $R^{16}$ für Wasserstoff oder Methyl stehen und gleich oder verschieden sind,

$R^{17}$ für Phenyl steht
und

$R^{18}$ für Carboxy, geradkettiges oder verzweigtes Alkoxycarbonyl mit bis zu 4 Kohlenstoffatomen steht oder
für eine Formel $-CH_2OH$ oder $-CONH_2$ steht,

gegebenenfalls in einer isomeren Form und deren Salze.

Außerdem wurde ein Verfahren zur Herstellung der erfindungsgemäßen Verbindungen der allgemeinen Formel (1) gefunden, dadurch gekennzeichnet, daß man
Verbindungen der allgemeinen Formel (II)

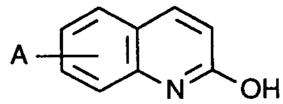 (II)

in welcher

A die oben angegebene Bedeutung hat,

zunächst mit Verbindungen der allgemeinen Formel (III)

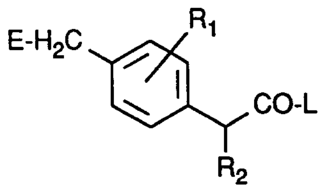 (III)

in welcher

$R^1$ und $R^2$ die oben angegebene Bedeutung haben,

E für eine typische Abgangsgruppe wie beispielsweise Chlor, Brom, Jod, Tosylat oder Mesylat, vorzugsweise für Brom steht,

und

L für geradkettiges oder verzweigtes ($C_1$-$C_4$)-Alkoxy steht,

in inerten Lösemitteln, in Anwesenheit einer Base und gegebenenfalls unter Schutzgasatmosphäre zu den Verbindungen der allgemeinen Formel (IV)

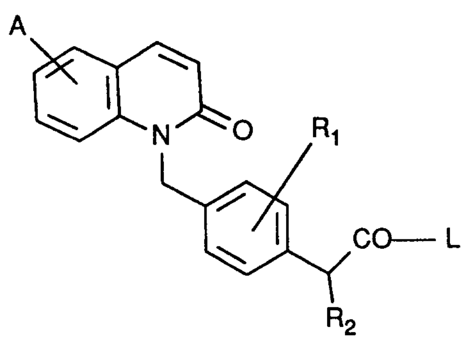 (IV)

in welcher

A, L, $R^1$ und $R^2$ die oben angegebene Bedeutung haben,

umsetzt,

und im Fall der Säuren ($R^3$ = OH) die Ester verseift,

und im Fall der Amide und Sulfonamide ausgehend von den Säuren, gegebenenfalls unter vorgeschalteter Aktivierung, in inerten Lösemitteln, gegebenenfalls in Anwesenheit einer Base und/oder Dehydratisierungsmittel mit Aminen und Phenylglycinolen der allgemeinen Formeln (V), (VI) und (VII)

$HNR^4R^5$ (V),

$HNR^6$-$CHR^7R^8$ (VI)

oder

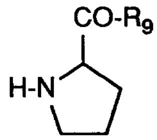

(VII)

in welchen

$R^4$, $R^5$, $R^6$, $R^7$, $R^8$ und $R^9$ die oben angegebene Bedeutung haben,

umsetzt,

und gegebenenfalls die Substituenten A und $R^1$ nach üblichen Methoden variiert,

und gegebenenfalls die Isomeren trennt, und im Fall der Herstellung der Salze mit einer entsprechenden Base oder Säure umsetzt,

und im Fall der Ester, ausgehend von den aktivierten Carbonsäuren, mit den entsprechenden Alkoholaten umsetzt.

Das erfindungsgemäße Verfahren kann durch folgendes Formelschema beispielhaft erläutert werden:

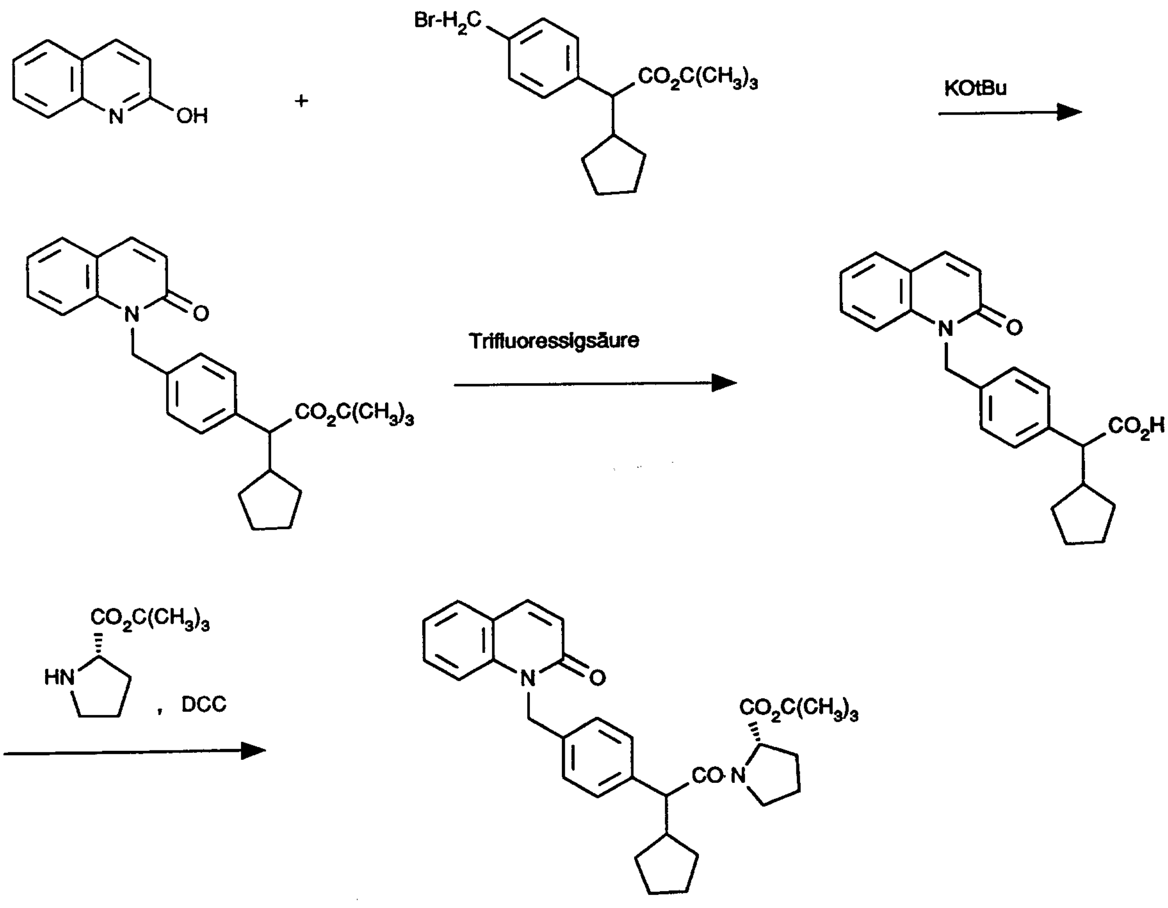


Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichiorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nikomethan. Ebenso ist es möglich, Gemische der genannten Lösemittel zu verwenden. Bevorzugt sind Dimethylformamid und Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate

wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat.

Im allgemeinen setzt man die Base in einer Menge von 0,05 mol bis 10 mol, bevorzugt von 1 mol bis 2 mol bezogen auf 1 mol der Verbindung der Formel (III) ein.

Das erfindungsgemäße Verfahren wird im allgemeinen in einem Temperaturbereich von -30 °C bis +100 °C, bevorzugt von -10 °C bis +60 °C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Hydrolyse die üblichen anorganischen Basen. Hierzu gehören bevorzugt Alkalihydroxide oder Erdalkalihydroxide wie beispielsweise Lithiumhydroxid, Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Lithiumhydroxid, Natriumhydroxid oder Kaliumhydroxid eingesetzt.

Als Lösermittel eignen sich für die Verseifung Wasser oder die für eine Hydrolyse üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Bevorzugt erfolgt die Hydrolyse mit Säuren wie beispielsweise Trifluoressigsäure, Essigsäure, Chlorwasserstoffsäure, Chlorwasserstoffsäure / Dioxan, Bromwasserstoffsäure, Methansulfonsäure, Schwefelsäure oder Perchlorsäure, besonders bevorzugt mit Trifluoressigsäure oder Chlorwasserstoffsäure / Dioxan.

Die Hydrolyse wird im allgemeinen in einem Temperaturbereich von 0 °C bis +100 °C, bevorzugt von +20 °C bis +80 °C, durchgeführt.

Im allgemeinen wird die Hydrolyse bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol, bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Säuren wie beispielsweise Chlorwasserstoffsäure, Bromwasserstoffsäure, Schwefelsäure, Phosphorsäure oder Trifluoressigsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden.

Die Amidierung und die Sulfoamidierung erfolgt im allgemeinen in einem der oben aufgeführten Lösemittel, vorzugsweise in Tetrahydrofuran oder Dichlormethan.

Die Amidierung oder Sulfoamidierung kann ausgehend von den entsprechenden Säuren der Verbindungen gegebenenfalls über die aktivierte Stufe der Säurehalogenide oder gemischten Anhydride, die aus den entsprechenden Säuren durch Umsetzung mit Thionylchlorid, Phosphortrichlorid, Phosphorpentachlorid, Phosphortribromid oder Oxalylchlorid oder Methansulfonsäurechlorid hergestellt werden können, verlaufen.

Die Amidierung oder Sulfoamidierung erfolgt im allgemeinen in einem Temperaturbereich von -50 °C bis +80 °C, vorzugsweise von -30 °C bis +20 °C, und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 5 mol, bezogen auf 1 mol der Verbindungen der allgemeinen Formel (V), (VI) und (VII) eingesetzt.

Als säurebindende Mittel für die Amidierung oder Sulfoamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[4.3.0]-nonen-5 (DBN) oder 1,8-Diazabicyclo-[5.4.0]undecen-5 (DBU) eingesetzt werden. Bevorzugt ist Triethylamin.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3'-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesterazid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der allgemeinen Formeln (II), (V), (VI) und (VII) sind an sich bekannt oder können nach üblichen Methoden hergestellt werden.

Die Verbindungen der allgemeinen Formel (III) sind teilweise bekannt oder neu und können dann beispielsweise hergestellt werden, indem man die entsprechenden 4-Methylverbindungen im Sinne einer Substitution, beispielsweise durch Halogenisierung in Anwenheit einer der oben aufgeführten Basen und/oder Hilfstoffe und eines der Lösemittel umsetzt.

Die Verbindungen der allgemeinen Formel (IV) sind neu und können beispielsweise, wie oben beschrieben, hergestellt werden.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretions-stimulierenden Effekte des Angiotensin II. Darüberhinaus inhibieren sie die Proliferation von glatten Muskelzellen.

Sie können deshalb in Arzneimitteln zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37 °C temperierter, Carbogen-begaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

Agonisten und ihre Standardkonzentrationen (Applikationsvolumen pro Einzelgabe = 100 µl):

| | | |
|---|---|---|
| KCl | 22,7;32,7;42,7;52,7 | mmol/l |
| L-Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infüsion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft Dazu wurde die rechte Nierenarterie mit einem Silberclip von 0,18 mm lichter Weite eingeengt Bei dieser Hypertonieform ist die Plasmareninaktivität in den ersten sechs Wochen nach dem Eingriff erhöht.

Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.

Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), $^3H$-Angiotensin II (3-5 nM), Test-Pufferlösung (50 mM Tris, pH 7,2,5 mM $MgCl_2$) sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert Danach werden die Zellen durch Serumentzug für 2 - 3 Tage synchronisiert und sodann mit Serum oder anderen Faktoren zum Wachstum angeregt Gleichzeitig werden Testverbindungen zugesetzt. Nach 16 - 20 Stunden wird 1 µCi $^3$H-Thymidin zugefügt und nach weiteren 4 Stunden der Einbau dieser Substanz in die TCA-präzipitierbare DNA der Zellen bestimmt. Zur Bestimmung der $IC_{50}$-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fallen die genannte obere Grenze überschritten werden muß. Im Falle der Applikation größerer Mengen kann es empfehlenswert sein, diese in mehreren Einzelgaben über den Tag zu verteilen.

Laufmittel

(A) Ether / Petrolether = 7:3
(B) Dichiormethan/Methanol = 9:1

Ausgangsverbindungen

Beispiel I

4-Methlyphenlyessigsäure-tert.butylester

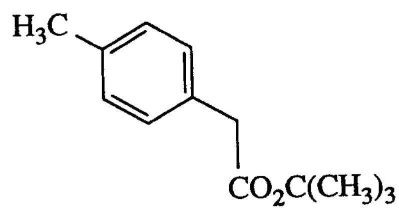

450 g (3 mol) 4-Methylphenylessigsäure, 1,13 1 (12 mol) tert.Butanol und 90 g (0,74 mol) Dimethylaminopyridin werden in 2 1 Dichlormethan gelöst. Nach Zugabe von 680 g (3,3 mol) Dicyclohexylcarbodiimid, gelöst in 400 ml Dichlormethan, wird 20 h bei 25 °C gerührt, der ausgefallene Harnstoff abgesaugt, mit 200 ml Dichlormethan gewaschen und die organische Phase je zweimal mit 500 ml 2 N Salzsäure und Wasser gewaschen. Die organische Phase wird eingeengt und destilliert. Ausbeute: 408 g (66% der Theorie) Siedepunkt: 73- 78 °C / 0,2 mm

Beispiel II

2-Cyclopentyl-2-(4-methylphenyl)essigsäure-tert.butylester

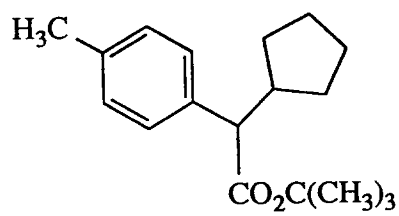

33,5 g (0,3 mol) Kalium-tert.butylat werden in 100 ml DMF unter Feuchtigkeitsausschluß bei 0 °C vorgelegt, und 51,6 g (0,25 mol) 4-Methylphenylessigsäuretert.butylester in 250 ml DMF zugetropft. Es wird 30 min bei 0 °C gerührt und 32,2 ml (0,3 mol) Cyclopentylbromid in 150 ml DMF bei 5-15 °C zugetropft und 20 h bei 25 °C gerührt. Nach Einengen wird der Rückstand zwischen Wasser/Diethylether verteilt, die Etherphase über Natriumsulfat getrocknet und eingeengt. Das Produkt kristallisiert aus.
Ausbeute: 67 g (97,5% der Theorie)
Erstaraungspunkt: 51 - 53 °C

Beispiel III

2-(4-Brommethyl-phenyl)-2-cyclopentyl-essigsäure-tert.butylester

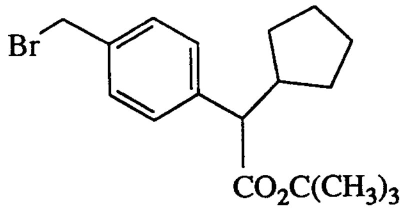

27,4 g (0,1 mol) 2-Cyclopentyl-2-(4-methylphenyl)-essigsäure-tert.butylester werden in 200 ml Tetrachlorkohlenstoff gelöst und zum Sieden erhitzt. Nach Zugabe von 0,82 g Azobisisobutyronitril werden 18,7 g (0,105 mol) N-Bromsuccinimid portionsweise zugegeben und anschließend 1 h refluxiert, auf 0 °C abgekühlt und vom Succinimid abfiltriert. Nach Einengen des Filtrates fällt das Produkt aus. Es wird mit Petrolether (40/60) gewaschen und getrocknet.
Ausbeute: 20 g (57% der Theorie)
Erstarrungspunkt: 73 - 76 °C

Herstellungsbeispiele

Beispiel 1

2-[4-(2-Oxochinolin-1-yl-methyl)phenyl]-2-cyclopentyl-essigsäure-tert.butylester

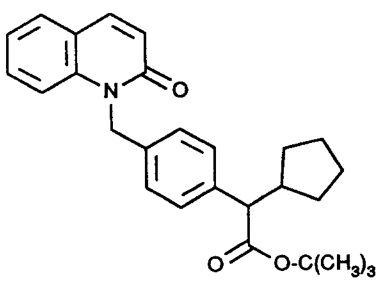

2,2 g (0,015 mol) 2-Hydroxychinolin und 50 g (0,014 mol) p-Brommethyl-2-cyclopentylessigsäure-tert.butylester werden unter Argonatmosphäre in 30 ml getrocknetem DMF gelöst und mit 1,9 g (0,015 mol) KOtBu, gelöst in 15 ml getr. DMF, tropfenweise unter Rühren bei Raumtemperatur versetzt. Man läßt über Nacht reagieren, dampft das Lösemittel im Vakuum ab (Rotavapor), nimmt den Rückstand unter gelindem Erwärmen in 50 ml Dichlormethan auf und wäscht 5 mal mit 20 ml Wasser. Nach Trocknen der organischen Phase mit $Na_2SO_4$ engt man auf ein Kleines Volumen ein und trennt den Rückstand säulenchromtographisch (Kieselgel 60, Laufmittel: Dichlormethan / Essigester = 100/5, $R_f$ = 0,6).
Ausbeute: 3,1 g (56,9% der Theorie) farbloses Öl.

Beispiel 2

2-[4-(2-Oxochinolin-1-yl-methyl)phenyl]-2-cyclopentyl-essigsäure

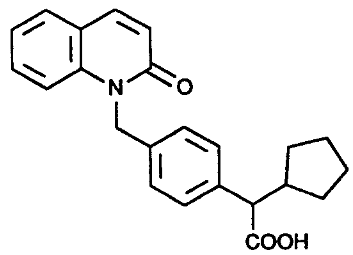

2,1 g (0,005 mol) der Verbindung aus Beispiel 1 werden mit 5 ml Trifluoressigsäure und 5 ml Dichlormethan 5 Stunden bei Raumtemperatur gerührt. Nach Einengen im Vakuum wird der Rückstand säulenchromatographiert (Kieselgel 60, Laufmittel: Toluol / Essigester / Eisessig = 80/10/5, $R_f$ = 0,5). Rektistallisation aus Diisopropylether.
Ausbeute: 1,5 g (83% der Theorie) farblose Kristalle
Fp.: 223 ° C

Beispiel 3

2-[4-(2-Oxochinolin-1-yl-methyl)phenyl]-2-cyclopentylessigsäure-(L-prolin-tert.burylester)

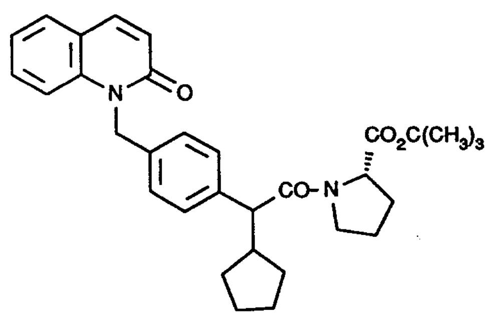

1,0 g (3 mmol) der Verbindung aus Beispiel 2 werden unter Rühren in 50 ml Dichlormethan bei Raumtemperatur unter Argon vorgelegt. Nach Zugabe von 0,9 ml (6 mmol) Triethylamin sowie einer Lösung von 0,69 g (4,5 mmol) 1-Hydroxy-1H-benzotriazol-monohydrat läßt man nach Abkühlen auf 0 ° C eine Lösung von 0,93 g (4,5 mmol) Dicyclohexylcarbodiimid in 15 ml Dichlormethan zutropfen. Nach 30 min Rühren wird bei gleicher Temperatur eine Lösung von 0,62 g (3,6 mmol) L-Prolin-tert.butylester in 10 ml Dichlormethan zugegeben. Nach 1 h läßt man auf Raumtemperatur kommen und rührt über Nacht. Zur Aufarbeitung wird mit Dichlormethan und Wasser versetzt, geschüttelt und die organische Phase abgetrennt. Die wäßrige Phase wird noch zweimal mit Dichlormethan extrahiert, und die vereinigten organischen Phasen werden über Natriumsulfat getrocknet, Filtriert, eingeengt und über Kieselgel 60 (Laufmittel: Petrolether / Essigester = 1:1) chromatographiert. Das Produkt wird anschließend in 100 ml Essigester suspendiert, 2 h bei 0 ° C gerührt, filtriert und eingeengt.
Ausbeute: 1,1 g (2,14 mmol)≙71% der Theorie
$R_f$ = 0,83 (Dichlormethan/Methanol = 9:1)

Beispiel 4

2-[4-(2-Oxochinolin-1-yl-methyl)phenyl]-2-cyclopentyl-essigsäure-L-prolin

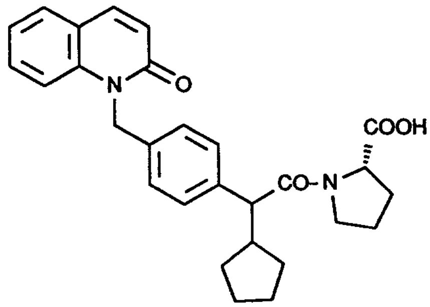

In Analogie zur Vorschrift des Beispiels 2 erfolgt die Herstellung der Titelverbindung durch Umsetzung von 1,1 g (2,1 mmol) der Verbindung aus Beispiel 2. Ausbeute: 0,94g (2,1 mmol)≙98% der Theorie
$R_f$ = 0,56 (Dichlormethan/Methanol = 9:1)

Beispiel 5

2-[4-(2-Oxochinolin-1-yl-methyl)phenyl]-2-cyclopentyl-essigsäure-(L-prolylphenyl-glycinol

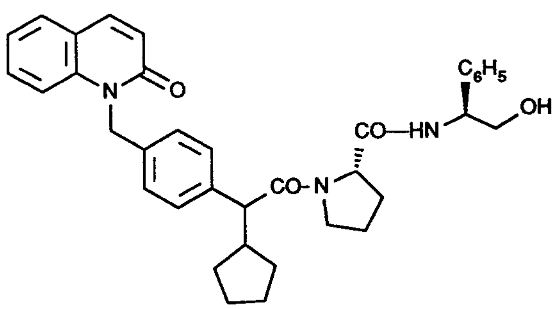

Die Herstellung der Titelverbindung erfolgt analog der Vorschrift zu Beispiel 3 durch Umsetzung von 0,46 g (1 mmol) der Verbindung aus Beispiel 4.
Ausbeute: 0,28 g (0,48 mmol)≙48% der Theorie
$R_f$ = 0,52 (Dichlormethan/Methanol = 9:1)
In Analogie zu den Vorschriften der Beispiele 1 bis 5 erfolgt die Herstellung der Verbindungen, die in Tabelle 1 aufgeführt sind:

Tabelle 1:

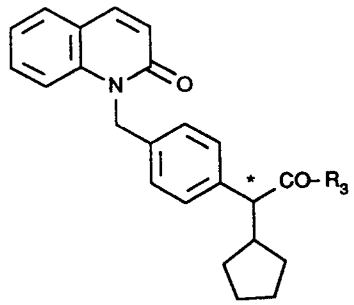

| Bsp.-Nr. | $R^3$ | * | $R_f$ (LM) | Herstellung analog Beispiel-Nr. |
|---|---|---|---|---|
| 6 | 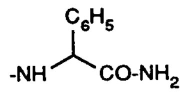 | dia | 0,31 (B) | 5 |
| 7 | 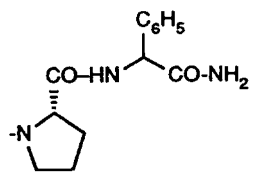 | dia | 0,46 (B) | 5 |

## Patentansprüche

1. 2-Oxochinolin-1-yl-methylphenylessigsäurederivate der allgemeinen Formel

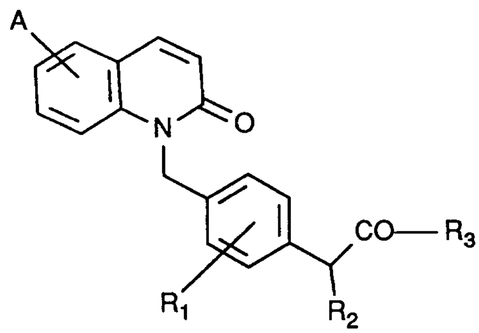

(I)

in welcher

A und $R^1$ gleich oder verschieden sind und für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano, Carboxy, Cycloalkyl mit 3 bis 8 Kohlenstoffatomen oder Phenyl stehen,

$R^2$ für Wasserstoff, Cycloalkyl mit 3 bis 8 Kohlenstoffatomen oder für geradkettiges oder verzweigtes Alkyl mit bis zu 8 Kohlenstoffatomen steht, das gegebenenfalls durch Cycloalkyl mit 3 bis 8 Kohlenstoffatomen substituiert ist,

$R^3$ für Hydroxy oder geradkettiges oder verzweigtes Alkoxy mit bis zu 8 Kohlenstoffatomen steht, oder für eine Gruppe der Formel

$-NR^4R^5$ ,

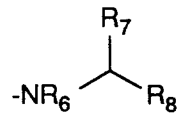

oder

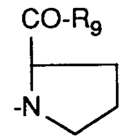

steht,
worin

$R^4$, $R^5$ und $R^6$ gleich oder verschieden sind und Wasserstoff, geradkettiges oder verzweigtes Alkyl mit bis zu 6 Kohienstoffatomen oder Phenyl bedeuten,
oder

$R^4$ die oben angegebene Bedeutung hat
und

$R^5$ eine Gruppe der Formel $-SO_2-R^{10}$ bedeutet,
worin

$R^{10}$ geradkettiges oder verzweigtes Alkyl mit bis zu 6 Kohlenstoffatomen bedeutet, das gegebenenfalls durch Phenyl substituiert ist, oder Trifluormethyl bedeutet
oder
Phenyl bedeutet, das gegebenenfalls durch geradkettiges oder verzweigtes Alkyl mit bis zu 4 Kohlenstoffatomen substituiert ist,

$R^7$ Phenyl bedeutet, das gegebenenfalls bis zu 2-fach gleich oder verschieden durch Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano, Carboxy, Cycloalkyl mit 3 bis 8 Kohlenstoffatomen oder Phenyl substituiert ist,

$R^8$ Carboxy oder geradkettiges oder verzweigtes Alkoxycarbonyl mit bis zu 6 Kohlenstoffatomen bedeutet, oder
eine Gruppe der Formel $-CH_2-OR^{11}$, $-CO-NR^{12}R^{13}$ oder $-CH_2NR^{12}R^{13}$ bedeutet,
worin

$R^{11}$ Wasserstoff, Phenyl oder geradkettiges oder verzweigtes Alkyl mit bis zu 6 Kohlenstoffatomen bedeutet
und

$R^{12}$ und $R^{13}$ die oben angegebene Bedeutung von $R^4$ und $R^5$ haben und mit diesen gleich oder verschieden sind,

$R^9$ Hydroxy, Benzyloxy oder geradkettiges oder verzweigtes Alkoxy mit bis zu 8 Kohlenstoffatomen bedeutet, oder
eine Gruppe der Formel
$-NR^{14}R^{15}$ oder

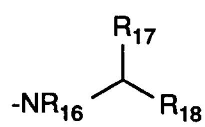

bedeutet,
worin

$R^{14}$ und $R^{15}$ die oben angegebene Bedeutung von $R^4$ und $R^5$ haben und mit dieser gleich oder verschieden sind,

$R^{16}$ die oben angegebene Bedeutung von $R^6$ hat und mit dieser gleich oder verschieden ist,

$R^{17}$ und $R^{18}$ jeweils die oben angegebene Bedeutung von $R^7$ bzw. $R^8$ haben und mit diesen gleich oder verschieden sind,

gegebenenfalls in einer isomeren Form und deren Salze.

**2.** 2-Oxochinotin-1-yl-methylphenylessigsäurederivate nach Anspruch 1, wobei

A und $R^1$ gleich oder verschieden sind und
für Wasserstoff, Fluor, Chlor, Brom, Trifluormethyl, Cyano oder geradkettiges oder

verzweigtes Alkyl, Alkoxy oder Alkoxycarbonyl mit jeweils bis zu 4 Kohlenstoffatomen stehen,

$R^2$ für Wasserstoff, Cyclopropyl, Cyclopentyl, Cyclohexyl oder Cycloheptyl steht, oder
für geradkettiges oder verzweigtes Alkyl mit bis zu 6 Kohlenstoffatomen steht, das gegebenenfalls durch Cyclopentyl, Cyclohexyl oder Cycloheptyl substituiert ist,

$R^3$ für Hydroxy oder geradkettiges oder verzweigtes Alkoxy mit bis zu 6 Kohlenstoffatomen steht, oder
für eine Gruppe der Formel
$-NR^4R^5$ ,

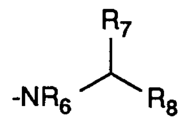 oder 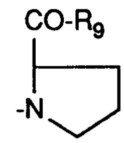

steht,
worin

$R^4$, $R^5$ und $R^6$ gleich oder verschieden sind und Wasserstoff oder geradkettiges oder verzweigtes Alkyl mit bis zu 4 Kohlenstoffatomen bedeuten,
oder

$R^4$ die oben angegebene Bedeutung hat
und

$R^5$ eine Gruppe der Formel $-SO_2-R^{10}$ bedeutet,
worin

$R^{10}$ geradkettiges oder verzweigtes Alkyl mit bis zu 4 Kohlenstoffatomen bedeutet, das gegebenenfalls durch Phenyl substituiert ist, oder Trifluormethyl bedeutet,
oder
Phenyl bedeutet, das gegebenenfalls durch geradkettiges oder verzweigtes Alkyl mit bis zu 3 Kohlenstoffatomen substituiert ist,

$R^7$ Phenyl bedeutet, das gegebenenfalls bis zu 2-fach gleich oder verschieden durch Fluor, Chlor, Brom, Trifluormethyl, Carboxy, geradkettiges oder verzweigtes Alkyl, Alkoxy oder Alkoxycarbonyl mit jeweils bis zu 4 Kohlenstoffatomen, Cyclopentyl, Cyclohexyl oder Phenyl substituiert ist,

$R^8$ Carboxy oder geradkettiges oder verzweigtes Alkoxycarbonyl mit bis zu 4 Kohlenstoffatomen bedeutet, oder
eine Gruppe der Formel $-CH_2-OR^{11}$, $-CO-NR^{12}R^{13}$ oder $-CH_2NR^{12}R^{13}$ bedeutet,
worin

$R^{11}$ Wasserstoff oder geradkettiges oder verzweigtes Alkyl mit bis zu 4 Kohlenstoffatomen bedeutet
und

$R^{12}$ und $R^{13}$ die oben angegebene Bedeutung von $R^4$ und $R^5$ haben und mit dieser gleich oder verschieden sind,

$R^9$ Hydroxy, Benzyloxy oder geradkettiges oder verzweigtes Alkoxy mit bis zu 6 Kohlenstoffatomen bedeutet, oder eine Gruppe der Formel
$-NR^{14}R^{15}$ oder

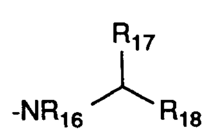

bedeutet,
worin

$R^{14}$ und $R^{15}$ die oben angegebene Bedeutung von $R^4$ und $R^5$ haben und mit dieser gleich oder verschieden sind,

| | |
|---|---|
| $R^{16}$ | die oben angegebene Bedeutung von $R^6$ hat und mit dieser gleich oder verschieden ist, |
| $R^{17}$ und $R^{18}$ | jeweils die oben angegebene Bedeutung von $R^7$ bzw. $R^8$ haben und mit diesen gleich oder verschieden sind, |

gegebenenfalls in einer isomeren Form und deren Salze.

**3.** 2-Oxochinolin-1-yl-methylphenylessigsäurederivate nach Anspruch 1,
wobei

| | |
|---|---|
| A und $R^1$ | gleich oder verschieden sind und für Wasserstoff, Fluor, Chlor, Brom, Trifluormethyl, Trifluormethoxy, Cyano oder Methyl stehen, |
| $R^2$ | für Wasserstoff, Cyclopropyl, Cyclopentyl, Cyclohexyl, Cycloheptyl oder für geradkettiges oder verzweigtes Alkyl steht, das gegebenenfalls durch Cyclopentyl oder Cyclohexyl substituiert ist, |
| $R^3$ | für Hydroxy oder geradkettiges oder verzweigtes Alkoxy mit bis zu 4 Kohlenstoffatomen steht, oder für eine Gruppe der Formel $-NR^4R^5$ , |

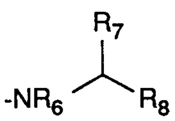

oder

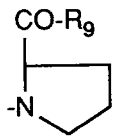

steht,
worin

| | |
|---|---|
| $R^4$, $R^5$ und $R^6$ | gleich oder verschieden sind und Wasserstoff oder Methyl bedeuten oder |
| $R^4$ | die oben angegebene Bedeutung hat und |
| $R^5$ | eine Gruppe der Formel $-SO_2-R^{10}$ bedeutet, worin |
| $R^{10}$ | Methyl, Benzyl oder p-Tolyl bedeutet, |
| $R^7$ | Phenyl bedeutet, das gegebenenfalls durch Fluor, Chlor, Brom, Trifluormethyl, Trifluormethoxy oder Methyl substituiert ist, |
| $R^8$ | Carboxy oder geradkettiges oder verzweigtes Alkoxycarbonyl mit bis zu 3 Kohlenstoffatomen bedeutet, oder eine Gruppe der Formel $-CH_2O-R^{11}$, $-CO-NR^{12}R^{13}$ oder $-CH_2NR^{12}R^{13}$ bedeutet, worin |
| $R^{11}$ | Wasserstoff oder geradkettiges oder verzweigtes Alkyl mit bis zu 3 Kohlenstoffatomen bedeutet und |
| $R^{12}$ und $R^{13}$ | die oben angegebene Bedeutung von $R^4$ und $R^5$ haben und mit dieser gleich oder verschieden sind, |
| $R^9$ | Hydroxy, Benzyloxy oder geradkettiges oder verzweigtes Alkoxy mit bis zu 4 Kohlenstoffatomen bedeutet, oder eine Gruppe der Formel $-NR^{14}R^{15}$ oder |

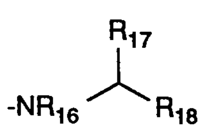

bedeutet,
worin

| | |
|---|---|
| $R^{14}$ und $R^{15}$ | die oben angegebene Bedeutung von $R^4$ und $R^5$ haben und mit dieser gleich |

oder verschieden sind,

$R^{16}$ die oben angegebene Bedeutung von $R^6$ hat und mit dieser gleich oder verschieden ist,

$R^{17}$ und $R^{18}$ jeweils die oben angegebene Bedeutung von $R^7$ bzw. $R^8$ haben und mit diesen gleich oder verschieden sind,

gegebenenfalls in einer isomeren Form und deren Salze.

**4.** 2-Oxochinotin-1-yl-methylphenylessigsäurederivate nach Anspruch 1, wobei

A und $R^1$ für Wasserstoff stehen,

$R^2$ für Cyclopentyl oder Cycloheptyl steht

und

$R^3$ für Hydroxy, geradkettiges oder verzweigtes Alkoxy mit bis zu 4 Kohlenstoffatomen oder

für eine Gruppe der Formel

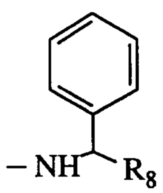

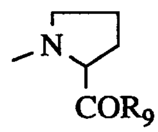

steht,

wobei

$R^8$ die $-CONH_2$-Gruppe bedeutet,

$R^9$ Hydroxy, geradkettiges oder verzweigtes Alkoxy mit bis zu 4 Kohlenstoffatomen bedeutet,

oder eine Gruppe der Formel

$-NR^{14}R^{15}$ oder

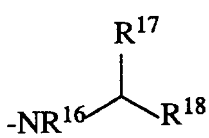

bedeutet,

worin

$R^{14}$, $R^{15}$, $R^{16}$ für Wasserstoff oder Methyl stehen und gleich oder verschieden sind,

$R^{17}$ für Phenyl steht

und

$R^{18}$ für Carboxy, geradkettiges oder verzweigtes Alkoxycarbonyl mit bis zu 4 Kohlenstoffatomen steht oder für eine Formel $-CH_2OH$ oder $-CONH_2$ steht,

gegebenenfalls in einer isomeren Form und deren Salze.

**5.** 2-Oxochinolin-1-yl-methylphenylessigsäurederivate nach den Ansprüchen 1 bis 4 zur therapeutischen Anwendung.

**6.** Verfahren zur Herstellung von 2-Oxochinolin-1-yl-methylphenylessigsäurederivaten nach Anspruch 1 bis 4, dadurch gekennzeichnet, daß man Verbindungen der allgemeinen Formel (II)

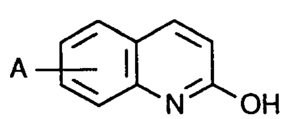

(II)

in welcher

A die oben angegebene Bedeutung hat,

zunächst mit Verbindungen der allgemeinen Formel (III)

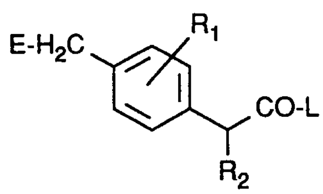

(III)

in welcher

$R^1$ und $R^2$ die oben angegebene Bedeutung haben,

E für eine typische Abgangsgruppe wie beispielsweise Chlor, Brom, Jod, Tosylat oder Mesylat, vorzugsweise für Brom steht.

und

L für geradkettiges oder verzweigtes ($C_1$-$C_4$)-Alkoxy steht,

in inerten Lösemitteln, in Anwesenheit einer Base und gegebenenfalls unter Schutzgasatmosphäre zu den Verbindungen der allgemeinen Formel (IV)

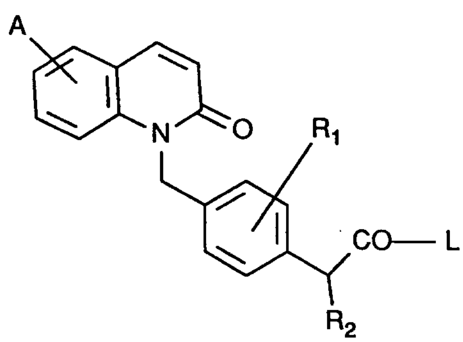

(IV)

in welcher

A, L, $R^1$ und $R^2$ die oben angegebene Bedeutung haben,

umsetzt,

und im Fall der Säuren ($R^3$ = OH) die Ester verseift,

und im Fall der Amide und Sulfonamide ausgehend von den Säuren, gegebenenfalls unter vorgeschalteter Aktivierung, in inerten Lösemitteln, gegebenenfalls in Anwesenheit einer Base und/oder Dehydratisierungsmittel mit Aminen und Phenylglycinolen der allgemeinen Formeln (V), (VI) und (VII)

$HNR^4R^5$ (V),

$HNR^6$-$CHR^7R^8$ (VI)

oder

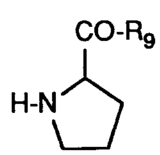

(VII)

in welchen

$R^4$, $R^5$, $R^6$, $R^7$, $R^8$ und $R^9$ die oben angegebene Bedeutung hahen,

umsetzt,

und gegebenenfalls die Substituenten A und $R^1$ nach üblichen Methoden variiert,

und gegebenenfalls die Isomeren trennt, und im Fall der Herstellung der Salze mit einer entsprechenden Base oder Säure umsetzt,
und im Fall der Ester, ausgehend von den aktivierten Carbonsäuren, mit den entsprechenden Alkoholaten umsetzt.

**7.** Arzneimittel enthaltend mindestens ein 2-Oxochinolin-1-yl-methylphenylessigsäurederivat nach Anspruch 1 bis 4.

**8.** Arzneimittel nach Anspruch 7 zur Behandlung von arterieller Hypertonie und Atherosklerose.

**9.** Verwendung von 2-Oxochinolin-1-yl-methylphenylessigsäurederivaten nach Anspruch 1 bis 4 zur Herstellung von Arzneimitteln.

**10.** Verwendung von 2-Oxochinolin-1-yl-methylphenylessigsäurederivaten nach Anspruch 1 bis 4 zur Herstellung von Arzneimitteln zur Behandlung von arterieller Hypertonie und Atherosklerose.

Europäisches Patentamt

# EUROPÄISCHER RECHERCHENBERICHT

Nummer der Anmeldung
EP 94 10 0366

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | EP-A-0 500 297 (FISONS PLC)<br>* Seite 11, Zeile 14-34 ; Beispiele 27, 31, usw. *<br>--- | 1,8 | C07D215/22<br>A61K31/47<br>C07D401/10 |
| A | EP-A-0 499 926 (BASF AG)<br>* Seite 10, Zeile 9-11; Ansprüche *<br>--- | 1 | |
| P,A | EP-A-0 569 795 (BAYER AG)<br>* Ansprüche *<br>----- | 1,8 | |

RECHERCHIERTE SACHGEBIETE (Int.Cl.5)

C07D
A61K

Der vorliegende Recherchenbericht wurde für alle Patentansprüche erstellt

| Recherchenort | Abschlußdatum der Recherche | Prüfer |
|---|---|---|
| DEN HAAG | 15. April 1994 | Van Bijlen, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)